(19) European Patent Office — Europäisches Patentamt / European Patent Office / Office européen des brevets

(11) **EP 4 617 498 A1**

(12) **EUROPEAN PATENT APPLICATION**

(43) Date of publication:
**17.09.2025 Bulletin 2025/38**

(21) Application number: **25162203.1**

(22) Date of filing: **06.03.2025**

(51) International Patent Classification (IPC):
*F04D 13/06* (2006.01)   *F04D 15/02* (2006.01)
*A61M 60/113* (2021.01)   *A61M 60/232* (2021.01)
*A61M 60/38* (2021.01)   *A61M 60/508* (2021.01)
*A61M 60/90* (2021.01)   *F04D 15/00* (2006.01)

(52) Cooperative Patent Classification (CPC):
**A61M 60/113; A61M 60/232; A61M 60/38;
A61M 60/508; A61M 60/90; F04D 13/06;
F04D 15/0088; F04D 15/0272;** A61M 2205/18;
A61M 2205/52

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **11.03.2024 CN 202410271788**

(71) Applicant: **ChinaBridge (Shenzen) Medical
Technology Co., Ltd.
Shenzhen, Guangdong 518102 (CN)**

(72) Inventor: **LI, Yijiang
Shenzhen Guangdong, 518102 (CN)**

(74) Representative: **Pfenning, Meinig & Partner mbB
Patent- und Rechtsanwälte
Joachimsthaler Straße 10-12
10719 Berlin (DE)**

(54) **METHOD AND DEVICE FOR PREDICTING BLOOD PUMP STATE, AND APPARATUS AND MEDIUM**

(57) A method for predicting a blood pump state, characterized in that a pump device is driven by a motor, includes collecting an average phase current of each phase of the motor in a current cycle, determining whether the average phase current of each phase of the motor is stable, determining whether the average phase current of each phase of the motor changes consistently in response to determining that the average phase current of each phase of the motor is unstable, determining whether the average phase current of each phase of the motor changes shows a cyclic change in response to determining that the average phase current of each phase of the motor changes consistently, and determining that a blood pump of the pump device is abnormal in response to determining that the average phase current of the motor changes shows the cyclic change.

FIG. 1

EP 4 617 498 A1

## Description

## Technical Field

The present invention relates to the technical field of medical devices, and in particular to a method and a device for predicting a blood pump state, an apparatus and a medium.

## Background Art

In clinical emergency treatment of critically ill patients with severe cardiopulmonary failure, extracorporeal membrane oxygenation (ECMO) is used to provide patients with continuous extracorporeal respiratory and circulatory support to gain more treatment time. The core parts of extracorporeal membrane oxygenation (ECMO) are artificial lungs (also known as membrane lungs or oxygenators) and artificial hearts (also known as blood pumps or power pumps). As the technologies related to artificial lungs and artificial hearts are becoming more and more sophisticated, ECMO can be maintained for a longer time, which also provides the prerequisite for ECMO to be used in the treatment of patients with cardiopulmonary failure. The existing ECMO mainly achieves blood flow by driving the blood pump to rotate. Blood pumps are usually disposable consumables. Since the ECMO system needs to be perfused with the patient's blood, the blood pump volume needs to be as small as possible in order to reduce the perfusion volume. In addition, because of its small size and the need to achieve the required flow, the blood pump often has a high speed when running. At the same time, since the use scenario thereof is often in the intensive care unit, the blood pump is often required to run smoothly and quietly.

When ECMO surgery is performed clinically, the difference in use scenarios may cause various conditions such as pipeline bending, bubbles, and a large number of thrombi in the blood pump after long-term operation. Even if there are no abnormalities, the differences in the recipients and the environment can cause the blood pump to wear to varying degrees. When the wear reaches a certain degree, it indicates that the pump has reached the limit of its service life. When the blood pump reaches the limit of its service life, a large deviation will occur. Large deviations may cause the temperature to rise and cause hemolysis.

Due to differences in the processing technology of the blood pump itself, the use environment factors, the patient's physical condition, etc., the service life of the blood pump can be different. Since the blood pump is a high-value-added consumable, the same blood pump will be used as much as possible during a surgery. The operation time of the blood pump is often relatively long, and its actual service life varies depending on the environment.

At present, there is no equipment that can clearly indicate the status of the blood pump. Clinical doctors and maintenance personnel of equipment manufacturers use experience to determine the status of the blood pump based on conditions such as flow and sound of the blood pump. For example, the existing method of detecting whether the blood pump is normal may judge the status of the blood pump by considering the relationship between flow and speed. However, this method is relatively rough. It only alarms when the flow and pump speed do not match, and it is impossible to distinguish it from the flow speed mismatch caused by factors such as pipeline blockage, folding, and bubble influence from the abnormality of the blood pump. In similar fields, by judging the current of the centrifugal pump, the method is relatively simple and cannot filter out some abnormal conditions. These methods are relatively inaccurate. This makes it difficult for the hospital to accurately predict the development of the disease, and it is difficult for the maintenance manufacturer to accurately predict the status of the equipment. At the same time, it also makes it difficult to determine the timing of blood pump replacement. Replacing the blood pump too early will cause waste of consumables, while replacing it too late will affect the patient's life safety.

## Summary

The embodiments of the present invention provide a method and a device for predicting a blood pump state, an apparatus and a medium, aiming to solve the problem that it is difficult in the existing technology to accurately determine the state of a blood pump.

In a first aspect, the embodiments of the present invention provide a method for predicting a blood pump state, the method comprising:

collecting an average phase current of each phase of the motor in a current cycle;
determining whether the average phase current of each phase of the motor is stable;
if the average phase current of each phase of the motor is unstable, determining whether the average phase current of each phase of the motor changes consistently;
if the average phase current of each phase of the motor changes consistently, determining whether the average phase current of each phase of the motor changes shows a cyclic change; and
if the average phase current of each phase of the motor changes shows the cyclic change, determining that a blood pump of the pump device is abnormal.

Through the above technical solution, it can accurately identify abnormal conditions of the blood pump and the system connected to the blood pump, and prompt the operator to deal with the abnormality in time, which improves the safety of ECMO equipment use and avoids the waste of consumables caused by premature replacement of the blood pump.

A further technical solution thereof is as follows: a tail end of the motor of the pump device is equipped with a Hall sensor, and the collecting of the average phase current of each phase of the motor in the current cycle comprises:

determining a starting point and an end point of the current cycle based on a Hall signal of the Hall sensor; and

determining the average phase current of each phase of the motor based on current sampling values collected in each phase of the motor during the current cycle.

Through the above technical solution, the starting point and the end point of the current cycle can be accurately determined, and thus the average value of the phase current of each phase of the motor can be accurately determined based on the current sampling values collected during the current cycle.

A further technical solution thereof is as follows: the determining whether the average phase current of each phase of the motor is stable comprises:

determining whether a current fluctuation value of the average phase current of each phase of the motor within a preset time range is greater than a preset current fluctuation threshold; and

if the current fluctuation value of the average phase current of each phase of the motor within the preset time range is greater than the preset current fluctuation threshold, determining that the average phase current of each phase of the motor is unstable.

Through the above technical solution, based on the set current fluctuation threshold, it is possible to accurately determine whether the average phase current value is stable.

A further technical solution thereof is as follows: the method further comprises:

if the average phase current of each phase of the motor changes inconsistently, determining whether a number of times the average phase current of each phase of the motor changing inconsistently is greater than a preset number of times threshold; and

if the number of times the average phase current of each phase of the motor changing inconsistently is greater than the preset number of times threshold, determining that the pump device has a hardware fault.

Through the above technical solution, it can accurately detect whether the pump device has a hardware failure, so that it can issue an alarm message to remind the user in time when a hardware failure occurs.

A further technical solution thereof is as follows:

prior to the determining whether the average phase current of each phase of the motor changes consistently, the method further comprises:

if the average phase current of each phase of the motor is unstable, determining whether a speed of the pump device is adjusted within the preset time range; and

if the speed of the pump device is not adjusted within the preset time range, executing the step of determining whether the average phase current of each phase of the motor changes consistently.

A further technical solution thereof is as follows: prior to the determining whether the average phase current of each phase of the motor changes shows the cyclic change, the method further comprises:

if the average phase current of each phase of the motor changes consistently, determining whether a flow fluctuation value of a flow of a pipeline where the pump device is located within the preset time range is greater than a preset flow fluctuation threshold;

if the average phase current of each phase of the motor changes consistently, determining whether the flow fluctuation value of the flow of the pipeline where the pump device is located within the preset time range is not greater than the preset flow fluctuation threshold, determining whether a bubble signal is detected in the pipeline where the pump device is located within the preset time range; and

if the bubble signal is not detected in the pipeline where the pump device is located within the preset time range, executing the step of determining whether the average phase current of each phase of the motor changes shows the cyclic change.

A further technical solution thereof is as follows: the method further comprises:

if the average phase current of each phase of the motor changes consistently, determining whether the flow fluctuation value of the flow of the pipeline where the pump device is located within the preset time range is greater than the preset flow fluctuation threshold, determining whether a pressure fluctuation value of the pipeline where the pump device is located within the preset time range is greater than a preset pressure fluctuation threshold;

if the pressure fluctuation value of the pipeline where the pump device is located within the preset time range is greater than the preset pressure fluctuation threshold, determining that the pipeline where the pump device is located is bent;

if the pressure fluctuation value of the pipeline where the pump device is located within the preset time range is not greater than the preset pressure fluctuation threshold, determining that the pipeline where the pump device is located is shaking; and

if the pipeline where the pump device is located is detected with a bubble signal within the preset time range, determining that a bubble is flowing in the pipeline where the pump device is located.

Through the above technical solution, it can accurately detect pipeline bending, shaking, bubbles, etc., avoid the interference of the above-mentioned situations on the blood pump status detection, and improve the accuracy of blood pump status detection.

A further technical solution thereof is as follows: the method further comprises:

if the average phase current of each phase of the motor is stable, determining whether the average phase current of each phase of the motor is greater than a preset no-load current;

if the average phase current of each phase of the motor is not greater than the preset no-load current, determining that the blood pump of the pump device has fallen off;

if the average phase current of each phase of the motor is greater than the preset no-load current, determining whether a flow of the pipeline where the pump device is located is stable; and

if the flow of the pipeline where the pump device is located is stable, determining that the blood pump of the pump device is normal.

Through the above technical solution, it can accurately detect the blood pump detachment or falling off, so that when the blood pump detaches, an alarm message can be issued to remind the user in time.

A further technical solution thereof is as follows: the method further comprises:

if the average phase current of each phase of the motor does not show a cyclic change, determining whether a number of turns when the average phase current of each phase of the motor is unstable is greater than a preset number of turns threshold; and

if the number of turns when the average phase current of each phase of the motor is unstable is greater than a preset number of turns threshold, determining that the blood pump of the motor is in a critical state between normal and abnormal.

Through the above technical solution, it can accurately detect whether the blood pump has reached the critical state between normal and abnormal, so as to issue an alarm message in time to remind the user to handle it.

In a second aspect, the embodiments of the present invention further provide a device for predicting a blood pump state the device comprises units for executing the method described above.

In a third aspect, the embodiments of the present invention further provide a computer device, the computer device comprises a memory and a processor, the memory stores a computer program, and the processor implements the method described above when executing the computer program.

In a fourth aspect, the embodiments of the present invention further provide a computer-readable storage medium, the storage medium stores a computer program, and when the computer program is executed by a processor, the method described above is implemented.

The embodiments of the present invention provide a method and a device for predicting a blood pump state, an apparatus and a storage medium. The method comprises: collecting an average phase current of each phase of the motor in a current cycle; determining whether the average phase current of each phase of the motor is stable; if the average phase current of each phase of the motor is unstable, determining whether the average phase current of each phase of the motor changes consistently; if the average phase current of each phase of the motor changes consistently, determining whether the average phase current of each phase of the motor changes shows a cyclic change; and if the average phase current of each phase of the motor changes shows the cyclic change, determining that a blood pump of the pump device is abnormal. Therefore, through the technical solution of the present invention, the abnormal conditions of the blood pump and the system connected to the blood pump can be accurately identified, and the operator can be prompted to deal with the abnormality in time, thereby improving the safety of the use of ECMO equipment. At the same time, it can also avoid the waste of consumables caused by premature replacement of the blood pump.

**Brief Description of the Drawings**

In order to more clearly illustrate the technical solutions of the embodiments of the present invention, the following will briefly introduce the drawings required for the description of the embodiments. Obviously, the drawings described below are some embodiments of the present invention. For a person of ordinary skill in the art, other drawings can be obtained based on these drawings without creative work.

FIG. 1 is a flow chart of a method for predicting a blood pump state provided in an embodiment of the present invention;
FIG. 2 is another flow chart of a method for predicting a blood pump state provided in an embodiment of the present invention;
FIG. 3 is a schematic block diagram of a device for predicting a blood pump state provided in an embodiment of the present invention;
FIG. 4 is a schematic block diagram of a computer device provided in an embodiment of the present invention.

## Description of the Embodiments

The following will clearly and fully describe the technical solutions in the embodiments of the present invention in conjunction with the drawings in the embodiments of the present invention. Obviously, the described embodiments are part of the embodiments of the present invention, not all of them. Based on the embodiments of the present invention, all other embodiments obtained by a person of ordinary skill in the art without creative work are within the scope of protection of the present invention.

It should be understood that when used in this specification and the appended claims, the terms "include" and "comprise" indicate the presence of the described features, wholes, steps, operations, elements and/or components, but do not exclude the presence or addition of one or more other features, wholes, steps, operations, elements, components and/or their collections.

It should also be understood that the terms used in this description of the present invention are only for the purpose of describing specific embodiments and are not intended to limit the present invention. As used in the specification of the present invention and the appended claims, the singular forms "a," "an" and "the" are intended to include the plural forms unless the context clearly indicates otherwise.

It should be further understood that the term "and/or" used in the present description and the appended claims refers to any combination of one or more of the associated listed items and all possible combinations, and includes these combinations.

As used in the present description and the appended claims, the term "if" can be interpreted as "when ..." or "once" or "in response to determination" or "in response to detection" depending on the context. Similarly, the phrase "if it is determined" or "if [the described condition or event] is detected" can be interpreted as meaning "once it is determined" or "in response to determination" or "once [the described condition or event] is detected" or "in response to detection of [the described condition or event]" depending on the context.

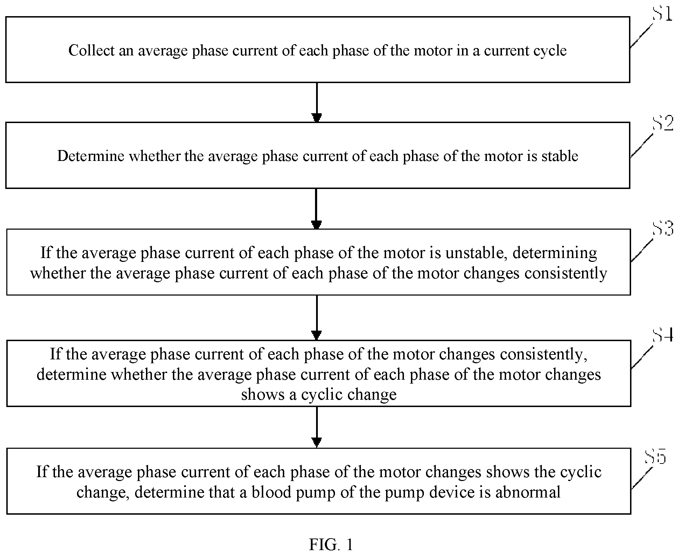

With reference to FIG. 1, an embodiment of the present invention provides a method for predicting a blood pump state. The method is used to predict the state of the blood pump of an ECMO device. In the present invention, the pump device is driven by a motor, and the blood pump is driven by the pump device. Specifically, the method for predicting a blood pump state includes the following steps:

S1. Collect an average phase current of each phase of the motor in a current cycle.

In a specific implementation, the blood pump of an ECMO device is driven by a pump device, which is driven by a three-phase motor. A three-phase motor is a type of motor that uses three-phase alternating current as a power source. A three-phase motor uses three current phases in a three-phase alternating current power source to generate a rotating magnetic field through mutually staggered current waveforms, thereby driving the rotor of the motor to rotate. A three-phase motor includes three phases in total. The waveform of the phase current of the three phases of the three-phase motor is a sine wave. One current cycle corresponds to a complete sine wave.

Specifically, in an embodiment of the present invention, the average values of the phase currents of the three phases of the three-phase motor in one current cycle are obtained respectively.

For example, in one embodiment, a tail end of the motor of the pump device is equipped with a Hall sensor, and the collecting of the average phase current of each phase of the motor in the current cycle comprises: determining a starting point and an end point of the current cycle based on a Hall signal of the Hall sensor; and determining the average phase current of each phase of the motor based on current sampling values collected in each phase of the motor during the current cycle.

In a specific implementation, in order to accurately determine the average value of the phase current of each phase of the motor, a complete sinusoidal wave cycle needs to be acquired, and identifying a complete cycle requires being able to identify the starting position and the end position of the cycle.

In the embodiments of the present invention, the Hall sensor is installed at the tail end of the three-phase motor. A complete cycle of the Hall sensor is equal to a complete cycle of the phase current. The sequence of the position numbers of the rotor detected by the Hall sensor is (5, 1, 3, 2, 6, 4). The moment when the detection starts with position 5 is the moment when the sine wave starts, and the moment when the position 4 ends is the moment when the sine wave ends. In this way, a complete cycle is obtained, and the average value of the phase current is calculated by the following formula:

$$I_a = \left( \sum_{n=1}^{N} a_n \right) / N$$ , where N is the number of samplings, $a_n$ is the current sampled at the nth time, and $I_a$ is the average value of the phase current.

This means performing the sum of the items from n=1 to N, which is then divided by the number of samplings, so as to obtain the average value of the sine wave, that is, the average phase current.

S2. Determine whether the average phase current of each phase of the motor is stable.

In a specific implementation, it is determined whether the average value of the phase current of each phase of the motor is stable, that is, whether the average phase current of all phases of the motor is stable. A stable average value of the phase current refers to that the average value of the phase current is relatively stable and does not fluctuate. The fluctuation of the phase current is often associated with the load change. Since the blood pump often runs smoothly and has a small overall volume, the stability of the operation of the blood pump is easy to change in the presence of pressure changes caused by pipe bending or wear of the blood pump, and a change in stability will further manifest as load fluctuation. Due to the high speed of the blood pump during operation, even a slight load fluctuation can easily cause changes in the three-phase current.

For example, in one embodiment, the determining whether the average phase current of each phase of the motor is stable comprises: determining whether a current fluctuation value of the average phase current of each phase of the motor within a preset time range is greater than a preset current fluctuation threshold; and if the current fluctuation value of the average phase current of each phase of the motor within the preset time range is greater than the preset current fluctuation threshold, determining that the average phase current of each phase of the motor is unstable. In the embodiments of the present invention, the current fluctuation threshold can be set by a person skilled in the art based on experience, and the present invention does not specifically limit it.

In a specific implementation, the preset time range can be set by a person skilled in the art, and the present invention does not specifically limit it. For example, the time for the motor to rotate one circle can be set as the preset time range. The preset time range includes multiple current cycles. The current fluctuation value refers to the difference between the maximum and minimum values of the average value of the phase current within the preset time range.

If the current fluctuation value of the phase current average value of any phase of the motor within the preset time range is greater than the preset current fluctuation threshold, it is determined that the phase current average value of each phase of the motor is unstable. If the current fluctuation value of the phase current average value of all phases of the motor within the preset time range is not greater than the preset current fluctuation threshold, it is determined that the phase current average value of each phase of the motor is stable.

S3. If the average phase current of each phase of the motor is unstable, determine whether the average phase current of each phase of the motor changes consistently.

In a specific implementation, if the average value of the phase current of each phase of the motor is unstable, it is determined whether the average value of the phase current of each phase of the motor changes consistently. The consistent change of the average value of the phase current herein refers to that the change trend of the average value of the phase current is consistent, that is, the average values of the phase current of each phase increase or decrease at the same time. If some of the average values of the phase currents of the phases increase while some of the average values of the phase currents of the phases decrease, it is considered that the average value of the phase current of each phase does not change consistently.

S4. If the average phase current of each phase of the motor changes consistently, determine whether the average phase current of each phase of the motor changes shows a cyclic change.

In a specific implementation, if the average value of the phase currents of each phase of the motor changes constantly, it is then determined whether the average value of the phase currents of each phase of the motor shows a cyclic changes. That is, it is determined whether the increase or decrease of the average value of the phase currents of each phase appears cyclically.

For example, in one embodiment, the number of pole pairs of the motor is 7, so there are 7 current sine waves when the motor rotates one circle, 6*7=42. Therefore, the time when the software detects 42 Hall signals through the Hall sensor is the time when the motor rotor rotates one circle. It is determined whether the position where the current abnormality occurs in the process of the motor rotating one circle appears 10 times in a row. If so, it indicates that there is a cyclic change. For example, if the average value of the second three-phase current in the first circle is unusually large, and the second three-phase current in each circle of the next 9 circles is also usually large, it is determined that there is a cyclic change.

S5. If the average phase current of each phase of the motor changes shows the cyclic change, determine that a blood pump of the pump device is abnormal.

In a specific implementation, if the average value of the phase current of each phase of the motor shows a cyclic change, it is determined that the blood pump of the pump device has an abnormality. In such a case, a high-level alarm message can be issued to indicate that the blood pump has an abnormality, so that the operator can know the abnormality of the blood pump in time and deal with it in time.

The technical solution of the embodiments of the present invention includes, for ECMO equipment, collecting an average phase current of each phase of the motor in a current cycle; determining whether the average phase current of each phase of the motor is stable; if the average phase current of each phase of the motor is unstable, determining whether the average phase current of each phase of the motor changes consistently; if the average phase current of each phase of the motor changes consistently, determining whether the average phase current of each phase of the motor changes shows a cyclic change; and if the average phase current of each phase of the motor changes shows the cyclic change, determining that a blood pump of the pump device is abnormal. Therefore, through the technical solution of the present invention, the abnormal conditions of the blood pump and the system connected to the blood pump can be accurately identified, and the operator can be prompted to deal with the abnormality in time, thereby improving the safety of the use of ECMO equipment. At the same time, it can also avoid the waste of consumables caused by premature replacement of the blood pump.

It should be noted that in the present invention, all set thresholds (for example, current fluctuation threshold, number threshold and lap threshold) are lower than the respective actual thresholds (the actual thresholds can be obtained through actual measurements). The purpose of such setting is to leave a certain amount of time for the operator to handle the abnormality of the blood pump after identifying it, or to ensure that the blood pump can still be used during the process of the operator discovering the abnormality and handling it, so as to ensure the safety of the use of the blood pump.

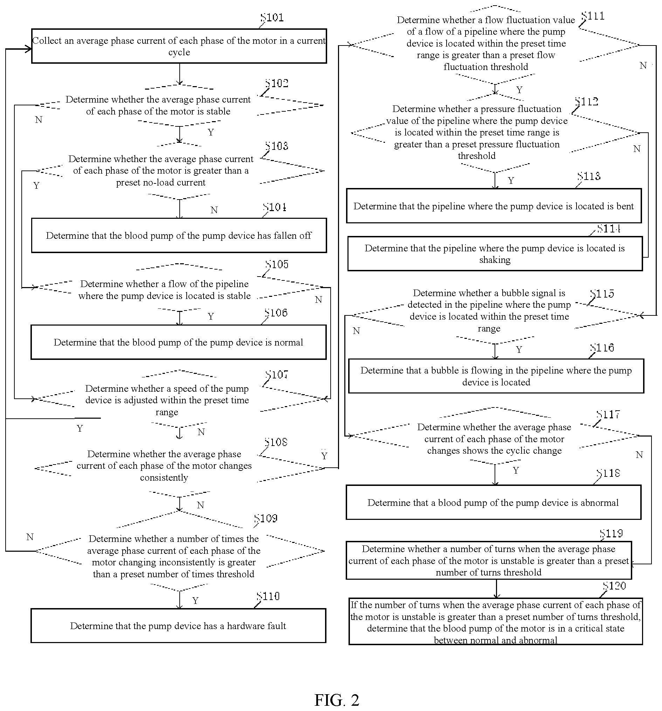

With reference to FIG. 2, an embodiment of the present invention provides a method for predicting a blood pump state, which can be used to predict the state of the blood pump of an ECMO device, and the method includes the following steps:

S101. Collect an average phase current of each phase of the motor in a current cycle.

In a specific implementation, the blood pump of the ECMO device can be driven by a three-phase motor. A three-phase motor is a type of motor that uses three-phase alternating current as a power source. A three-phase motor uses three current phases in a three-phase alternating current power source to generate a rotating magnetic field through mutually staggered current waveforms, thereby driving the rotor of the motor to rotate. A three-phase motor includes three phases in total. The waveform of the phase current of the three phases of the three-phase motor is a sine wave. One current cycle corresponds to a complete sine wave.

Specifically, in an embodiment of the present invention, the average values of the phase currents of the three phases of the three-phase motor in one current cycle are obtained respectively.

In one embodiment, a tail end of the motor of the pump device is equipped with a Hall sensor, and the collecting of the average phase current of each phase of the motor in the current cycle comprises: determining a starting point and an end point of the current cycle based on a Hall signal of the Hall sensor; and determining the average phase current of each phase of the motor based on current sampling values collected in each phase of the motor during the current cycle.

In a specific implementation, in order to accurately determine the average value of the phase current of each phase of the motor, a complete sinusoidal wave cycle needs to be acquired, and identifying a complete cycle requires being able to identify the starting position and the end position of the cycle.

In the embodiments of the present invention, the Hall sensor is installed at the tail end of the three-phase motor. A complete cycle of the Hall sensor is equal to a complete cycle of the phase current. The sequence of the position numbers of the rotor detected by the Hall sensor is (5, 1, 3, 2, 6, 4). The moment when the detection starts with position 5 is the moment when the sine wave starts, and the moment when the position 4 ends is the moment when the sine wave ends. In this way, a complete cycle is obtained, and the average value of the phase current is calculated by the following formula:

$$I_a = \left( \sum_{n=1}^{N} a_n \right) / N$$

, where N is the number of samplings, $a_n$ is the current sampled at the nth time, and $I_a$ is the average value of the phase current.

This means performing the sum of the items from n=1 to N, which is then divided by the number of samplings, so as to obtain the average value of the sine wave, that is, the average phase current.

S102. Determine whether the average phase current of each phase of the motor is stable.

In a specific implementation, it is determined whether the average value of the phase current of each phase of the motor is stable, that is, whether the average phase current of all phases of the motor is stable. A stable average value of the phase current refers to that the average value of the phase current is relatively stable and does not fluctuate.

In one embodiment, the determining whether the average phase current of each phase of the motor is stable comprises: determining whether a current fluctuation value of the average phase current of each phase of the motor within a preset time range is greater than a preset current fluctuation threshold; and if the current fluctuation value of the average phase current of each phase of the motor within the preset time range is greater than the preset current fluctuation threshold, determining that the average phase current of each phase of the motor is unstable.

In a specific implementation, the preset time range can be set by a person skilled in the art, and the present invention does not specifically limit it. For example, the time for the motor to rotate one circle can be set as the preset time range. The preset time range includes multiple current cycles. The current fluctuation value refers to the difference between the maximum and minimum values of the average value of the phase current within the preset time range.

If the current fluctuation value of the phase current average value of any phase of the motor within the preset time range is greater than the preset current fluctuation threshold, it is determined that the phase current average value of each phase of the motor is unstable. If the current fluctuation value of the phase current average value of all phases of the motor within the preset time range is not greater than the preset current fluctuation threshold, it is determined that the phase current average value of each phase of the motor is stable.

S103. If the average phase current of each phase of the motor is stable, determine whether the average phase current of each phase of the motor is greater than a preset no-load current.

In a specific implementation, if the average value of the phase current of each phase of the motor is stable, it is further determined whether the average value of the phase current of each phase of the motor is greater than the preset no-load current. The no-load current herein refers to the average value of the phase current of each phase when the motor is unloaded. The no-load current is pre-determined and stored.

S104. If the average phase current of each phase of the motor is not greater than the preset no-load current, determine that the blood pump of the pump device has fallen off (i.e., detached from the motor).

In a specific implementation, if the average value of the phase current of each phase of the motor is not greater than the preset no-load current, it means that the motor is in a no-load state, and at this time, it is determined that the blood pump of the pump device has fallen off. A blood pump falling off prompt message is then issued to prompt the operator to handle the issue in time.

S105. If the average phase current of each phase of the motor is greater than the preset no-load current, determine whether a flow of the pipeline where the pump device is located is stable.

In a specific implementation, if the average value of the phase current of each phase of the motor is greater than the preset no-load current, it is then determined whether the flow of the pipeline where the pump device is located is stable.

The flow can be collected by a flow sensor. The method for determining whether the flow is stable is as follows: determine whether the flow fluctuation value of the flow within the preset time range is greater than the preset flow fluctuation threshold; if the flow fluctuation value of the flow within the preset time range is greater than the preset flow fluctuation threshold, determine that the flow of the pipeline where the pump device is located is unstable; tf the flow fluctuation value of the flow within the preset time range is not greater than the preset flow fluctuation threshold, determine that the flow of the pipeline where the pump device is located is stable. The flow fluctuation value is equal to the difference between the maximum flow value and the minimum flow value within the preset time range. The flow fluctuation threshold can be set by a person skilled in the art, and the present invention does not specifically limit it.

S106. If the flow of the pipeline where the pump device is located is stable, determine that the blood pump of the pump device is normal.

In a specific implementation, if the flow of the pipeline in which the pump device is located is stable, it is determined that the blood pump of the pump device is normal.

If the flow of the pipeline in which the pump device is located is unstable, then go to the step of determining whether the rotation speed of the pump device is adjusted within the preset time range.

S107. If the average phase current of each phase of the motor is unstable, determine whether a speed of the pump device is adjusted within the preset time range.

In a specific implementation, if the average value of the phase current of each phase of the motor is unstable, it is then determined whether the speed of the pump device is adjusted within the preset time range. Specifically, it is determined whether a speed adjustment instruction is received within the preset time range. If so, it means that the speed of the pump device is adjusted within the preset time range, and the adjustment may be an increase in speed or a decrease in speed.

If the rotation speed of the pump device is adjusted within the preset time range, go to step S101.

S108. If the speed of the pump device is not adjusted within the preset time range, determine whether the average phase current of each phase of the motor changes consistently.

In a specific implementation, if the average value of the phase current of each phase of the motor is unstable, it is then determined whether the average value of the phase current of each phase of the motor changes consistently. The consistent change of the average value of the phase current means that the change trend of the average value of the phase current is consistent, that is, the average value of the phase current of each phase increases or decreases at the same time, and the change size is the same. If some average value of the phase currents of phases increase, while others decrease, it is considered that the average value of the phase current of each phase does not change consistently.

The phases of the three-phase current of a motor are usually interrelated, and there is a certain phase relationship between them. In normal operation, the waveforms of the three-phase currents are usually similar, so their changing trends are consistent. But in some cases, such as problems caused by system hardware failures, the three-phase currents may change inconsistently.

S109. If the average phase current of each phase of the motor changes inconsistently, determine whether a number of times the average phase current of each phase of the motor changing inconsistently is greater than a preset number of times threshold.

In a specific implementation, if the average values of the phase currents of the various phases of the motor change inconsistently, it is then determined whether the number of times the average values of the phase currents of the various phases of the motor change inconsistently is greater than a preset number of times threshold. For example, if the average value of the phase current of only one phase increases, and the average values of the phase currents of the other two phases remain unchanged, it is considered that the average value changes inconsistently. The number of times threshold can be set by a person skilled in the art based on experience, and the present invention does not specifically limit it.

Specifically, in addition to hardware failures, some abnormal interference situations can also cause inconsistent changes in the average value of phase currents. However, abnormal interference situations usually only cause a small number of inconsistencies, and they are usually discontinuous. Therefore, in the present invention, it is determined whether the number of consecutive inconsistencies in the average value of phase currents of each phase of the motor is greater than a preset number of times threshold, so that the above-mentioned abnormal interference situations can be excluded.

S110. If the number of times the average phase current of each phase of the motor changing inconsistently is greater than the preset number of times threshold, determine that the pump device has a hardware fault.

In a specific implementation, if the number of times that the average value of the phase current of each phase of the motor changes inconsistently and continuously exceeds the preset number of times threshold, it is determined that the pump device has a hardware fault. The hardware fault is caused by the board or the motor, such as the resistance, voltage, and device abnormality of the board, which may cause the device to affect the phase current. The number of times threshold can be set by a person skilled in the art, and the present invention does not specifically limit it. For example, it is set to 10.

S111. If the average phase current of each phase of the motor changes consistently, determine whether a flow fluctuation value of a flow of a pipeline where the pump device is located within the preset time range is greater than a preset flow fluctuation threshold.

In a specific implementation, if the average value of the phase currents of each phase of the motor changes consistently, it is further determined whether the flow fluctuation value of the flow of the pipeline where the pump device is located within the preset time range is greater than the preset flow fluctuation threshold. The flow value can be collected by a flow sensor. The flow fluctuation value is equal to the difference between the maximum flow value and the minimum flow value within the preset time range.

S112. If the average phase current of each phase of the motor changes consistently, determining whether the flow fluctuation value of the flow of the pipeline where the pump device is located within the preset time range is greater than the preset flow fluctuation threshold, determine whether a pressure fluctuation value of the pipeline where the pump device is located within the preset time range is greater than a preset pressure fluctuation threshold.

In a specific implementation, the flow value may change due to the bending and shaking of the pipeline, which can in turn affect the phase current of the motor, and lead to misjudgment. Therefore, in order to improve the accuracy of the blood pump state prediction, if the flow fluctuation value of the flow of the pipeline where the pump device is located within the preset time range is greater than the preset flow fluctuation threshold, then it is further determined whether the pressure fluctuation value of the pressure of the pipeline where the pump device is located within the preset time range is greater than the preset pressure fluctuation threshold.

The pressure fluctuation threshold can be set by a person skilled in the art, and the present invention does not specifically limit it.

S113. If the pressure fluctuation value of the pipeline where the pump device is located within the preset time range is greater than the preset pressure fluctuation threshold, determine that the pipeline where the pump device is located is bent.

In a specific implementation, the pressure fluctuation when the pipeline is bent will be greater than the pressure fluctuation when the pipeline is shaken. Therefore, if the pressure fluctuation value of the pressure of the pipeline where the pump device is located within the preset time range is greater than the preset pressure fluctuation threshold, it is determined that the pipeline where the pump device is located is bent. At this time, a pipeline bending alarm message is issued to prompt the user to handle the issue in time. For example, since the bending of the pipeline is a more serious situation, an alarm message of "the pipeline is bent/squeezed" can be issued on the display panel, and an alarm prompt sound can be issued at the same time to prompt the user to handle the issue in time.

S114. If the pressure fluctuation value of the pipeline where the pump device is located within the preset time range is not greater than the preset pressure fluctuation threshold, determine that the pipeline where the pump device is located is shaking.

In a specific implementation, if the pressure fluctuation value of the pressure of the pipeline where the pump device is located within the preset time range is not greater than the preset pressure fluctuation threshold, it is determined that the pipeline where the pump device is located is shaking. Since shaking is a minor abnormality, only prompt information is issued in the present invention, and no alarm information is issued. The prompt information can also be set not to be displayed.

Through the above pressure detection process, the interference of pipeline bending, shaking, etc. on the prediction of the blood pump state can be eliminated, thereby improving the accuracy.

S115. If the flow fluctuation value of the flow of the pipeline where the pump device is located within the preset time range is not greater than the preset flow fluctuation threshold, determine whether a bubble signal is detected in the pipeline where the pump device is located within the preset time range.

In a specific implementation, the generation of bubbles will cause the flow rate to change, which will in turn affect the phase current of the motor, and this may lead to misjudgment. Therefore, in order to improve the accuracy of the blood pump state prediction, if the flow fluctuation value of the flow rate of the pipeline where the pump device is located within the preset time range is not greater than the preset flow fluctuation threshold, then it is determined whether the pipeline where the pump device is located is detected with a bubble signal within the preset time range. The bubble signal can be detected by a bubble sensor configured on the pipeline, which is not specifically limited by the present invention.

S116. If the pipeline where the pump device is located is detected with a bubble signal within the preset time range, determine that a bubble is flowing in the pipeline where the pump device is located.

In a specific implementation, if the pipeline where the pump device is located is detected with a bubble signal within the preset time range, it is determined that a bubble(s) is flowing through the pipeline where the pump device is located, and a pipeline bubble alarm message is issued at this time to prompt the user to handle the issue in time.

Through the above-mentioned bubble detection process, the interference of bubbles on the blood pump state prediction can be eliminated, thereby improving the accuracy.

S117. If the bubble signal is not detected in the pipeline where the pump device is located within the preset time range, determine whether the average phase current of each phase of the motor changes shows the cyclic change.

In a specific implementation, if the average value of the phase currents of each phase of the motor changes constantly, it is then determined whether the average value of the phase currents of each phase of the motor shows cyclic changes. That is, it is determined whether the increase or decrease of the average value of the phase currents of each phase appears cyclically.

For example, in one embodiment, the number of pole pairs of the motor is 7, so there are 7 current sine waves when the motor rotates one circle, 6*7=42. Therefore, the time when the software detects 42 Hall signals through the Hall sensor is the time when the motor rotor rotates one circle. It is determined whether the position where the current abnormality occurs in the process of the motor rotating one circle appears 10 times in a row. If so, it indicates that there is a cyclic change. For example, if the average value of the second three-phase current in the first circle is unusually large, and the second three-phase current in each circle of the next 9 circles is also unusually large, it is determined that there is a cyclic change.

S118. If the average phase current of each phase of the motor changes shows the cyclic change, determine that a blood pump of the pump device is abnormal.

In a specific implementation, if the average value of the phase current of each phase of the motor shows a cyclic change, it is determined that the blood pump of the pump device has an abnormality. In such a case, a high-level alarm message can be issued to indicate that the blood pump has an abnormality, so that the operator can know the abnormality of the blood pump in time and deal with the issue in time.

S119. If the average phase current of each phase of the motor does not show a cyclic change, determine whether a number of turns when the average phase current of each phase of the motor is unstable is greater than a preset number of turns threshold.

In a specific implementation, one rotation of the motor corresponds to multiple current cycles. For example, in one embodiment, the number of pole pairs of the motor is 7, so one rotation of the motor has 7 current sine waves, that is, 7 current cycles. In one rotation of the motor, as long as the average value of the phase current in any current cycle is unstable, it is considered that the motor has an unstable phase current average value in this rotation.

Specifically, the threshold of the number of turns can be set by a person skilled in the art based on experience, and the present invention does not specifically limit it. For example, it can be set to 5 turns, that is, the number of turns in which the average value of the phase current of the motor is unstable is greater than five turns.

S120. If the number of turns when the average phase current of each phase of the motor is unstable is greater than a preset number of turns threshold, determine that the blood pump of the motor is in a critical state between normal and abnormal.

In a specific implementation, if the number of turns when the average phase current value of the motor is unstable is greater than a preset number of turns threshold, the blood pump of the motor is determined to be in a critical state between normal and abnormal. This indicates that the blood pump is about to fail, and an alarm message can be issued to prompt the operator to handle the issue in time.

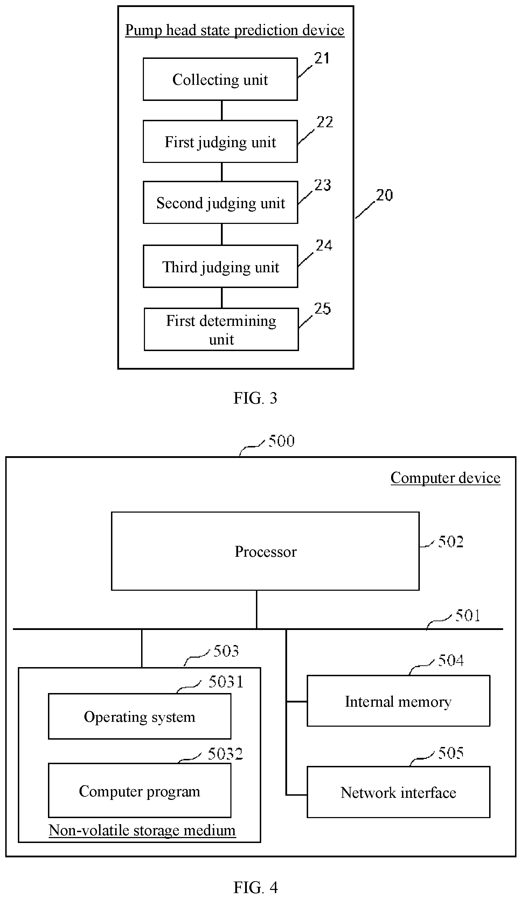

With reference to FIG. 3, FIG. 3 is a schematic block diagram of a device 20 for predicting a blood pump state provided in an embodiment of the present invention. Corresponding to the above method for predicting a blood pump state, the present invention further provides a device 20 for predicting a blood pump state. The device 20 for predicting a blood pump state includes certain units for executing the above method for predicting a blood pump state. The device 20 for predicting a blood pump state can be configured in a desktop computer, a tablet computer, a laptop computer, and other terminals. Specifically, the device 20 for predicting a blood pump state includes:

A collecting unit 21, which is configured to collect an average phase current of each phase of the motor in a current cycle;
A first judging unit 22, which is configured to determine whether the average phase current of each phase of the motor is stable;
A second judging unit 23, which is configured to, if the average phase current of each phase of the motor is unstable, determine whether the average phase current of each phase of the motor changes consistently;

A third judging unit 24, which is configured to, if the average phase current of each phase of the motor changes consistently, determine whether the average phase current of each phase of the motor changes shows a cyclic change; and

A first determining unit 25, which is configured to, if the average phase current of each phase of the motor changes shows the cyclic change, determining that a blood pump of the pump device is abnormal.

A further technical solution thereof is as follows: a tail end of the motor of the pump device is equipped with a Hall sensor, and the collecting of the average phase current of each phase of the motor in the current cycle comprises:

determining a starting point and an end point of the current cycle based on a Hall signal of the Hall sensor; and

determining the average phase current of each phase of the motor based on current sampling values collected in each phase of the motor during the current cycle.

A further technical solution thereof is as follows: the determining whether the average phase current of each phase of the motor is stable comprises:

determining whether a current fluctuation value of the average phase current of each phase of the motor within a preset time range is greater than a preset current fluctuation threshold; and

if the current fluctuation value of the average phase current of each phase of the motor within the preset time range is greater than the preset current fluctuation threshold, determining that the average phase current of each phase of the motor is unstable.

A further technical solution thereof is as follows: the device 20 for predicting a blood pump state further includes:

A fourth judging unit, which is configured to, if the average phase current of each phase of the motor changes inconsistently, determine whether a number of times the average phase current of each phase of the motor changing inconsistently is greater than a preset number of times threshold; and

A second determining unit, which is configured to, if the number of times the average phase current of each phase of the motor changing inconsistently is greater than the preset number of times threshold, determine that the pump device has a hardware fault.

A further technical solution thereof is as follows: the device 20 for predicting a blood pump state further includes:

A fifth judging unit, which is configured to, if the average phase current of each phase of the motor is unstable, determine whether a speed of the pump device is adjusted within the preset time range; and

The second judging unit, which is configured to, if the speed of the pump device is not adjusted within the preset time range, execute the step of determining whether the average phase current of each phase of the motor changes consistently.

A further technical solution thereof is as follows: the device 20 for predicting a blood pump state further includes:

A sixth judging unit, which is configured to, if the average phase current of each phase of the motor changes consistently, determine whether a flow fluctuation value of a flow of a pipeline where the pump device is located within the preset time range is greater than a preset flow fluctuation threshold;

A seventh judging unit, which is configured to, if the average phase current of each phase of the motor changes consistently, determining whether the flow fluctuation value of the flow of the pipeline where the pump device is located within the preset time range is not greater than the preset flow fluctuation threshold, determining whether a bubble signal is detected in the pipeline where the pump device is located within the preset time range; and

The third judging unit, which is configured to, if the bubble signal is not detected in the pipeline where the pump device is located within the preset time range, execute the step of determining whether the average phase current of each phase of the motor changes shows the cyclic change.

A further technical solution thereof is as follows: the device 20 for predicting a blood pump state further includes:

An eighth judging unit, which is configured to, if the average phase current of each phase of the motor changes consistently, determining whether the flow fluctuation value of the flow of the pipeline where the pump device is located within the preset time range is greater than the preset flow fluctuation threshold, determine whether a pressure fluctuation value of the pipeline where the pump device is located within the preset time range is greater than a preset pressure fluctuation threshold;

A third determining unit, which is configured to, if the pressure fluctuation value of the pipeline where the pump device is located within the preset time range is greater than the preset pressure fluctuation threshold, determine that the pipeline where the pump device is located is bent;

A fourth determining unit, which is configured to, if the pressure fluctuation value of the pipeline where the pump device is located within the preset time range is not greater than the preset pressure fluctuation threshold, determine that the pipeline where the pump device is located is shaking; and

A fifth determining unit, which is configured to, if the pipeline where the pump device is located is detected with a bubble signal within the preset time range, determine that a bubble is flowing in the pipeline where the pump device is located.

A further technical solution thereof is as follows: the device 20 for predicting a blood pump state further includes:

A ninth judging unit, which is configured to, if the average phase current of each phase of the motor is stable, determine whether the average phase current of each phase of the motor is greater than a preset no-load current;

A sixth determining unit, which is configured to, if the average phase current of each phase of the motor is not greater than the preset no-load current, determine that the blood pump of the pump device has fallen off;

A tenth judging unit, which is configured to, if the average phase current of each phase of the motor is greater than the preset no-load current, determine whether a flow of the pipeline where the pump device is located is stable; and

A seventh determining unit, which is configured to, if the flow of the pipeline where the pump device is located is stable, determine that the blood pump of the pump device is normal.

A further technical solution thereof is as follows: the device 20 for predicting a blood pump state further includes:

An eleventh judging unit, which is configured to, if the average phase current of each phase of the motor does not show a cyclic change, determine whether a number of turns when the average phase current of each phase of the motor is unstable is greater than a preset number of turns threshold; and

An eighth determining unit, which is configured to, if the number of turns when the average phase current of each phase of the motor is unstable is greater than a preset number of turns threshold, determine that the blood pump of the motor is in a critical state between normal and abnormal.

It should be noted that a person skilled in the art can clearly understand that the specific implementation process of the above-mentioned device 20 for predicting a blood pump state and each unit thereof can refer to the corresponding description in the above-mentioned method embodiments. For the convenience and simplicity of description, they will not be repeated herein.

The above-mentioned device 20 for predicting a blood pump remaining available time of a centrifugal pump can be implemented in the form of a computer program, and the computer program can be run on a computer device as shown in FIG 4.

With reference to FIG. 4, FIG. 4 is a schematic block diagram of a computer device provided in an embodiment of the present invention.

The computer device 500 may be a terminal or a server, where the terminal may be an electronic device with communication function such as a smart phone, a tablet computer, a laptop computer, a desktop computer, a personal digital assistant, and a wearable device etc. The server may be an independent server or a server cluster composed of multiple servers.

The computer device 500 includes a processor 502, a memory, and a network interface 505 connected via a system bus 501, where the memory may include a non-volatile storage medium 503 and an internal memory 504.

The non-volatile storage medium 503 may store an operating system 5031 and a computer program 5032. When the computer program 5032 is executed, the processor 502 may execute a method for predicting a blood pump state.

The processor 502 is configured to provide computing and control capabilities to support the operation of the entire computer device 500.

The internal memory 504 provides an environment for the operation of the computer program 5032 in the non-volatile storage medium 503. When the computer program 5032 is executed by the processor 502, the processor 502 can execute a method for predicting a blood pump state.

The network interface 505 is used for network communication with other devices. a person skilled in the art can understand that the above structure is only a block diagram of a part of the structure related to the scheme of the present application, and does not constitute a limitation on the computer device 500 to which the scheme of the present application is applied. The specific computer device 500 may include more or less components than those shown in the figure, or combine certain components, or have different component arrangements.

In this case, the processor 502 is configured to run the computer program 5032 stored in the memory to implement the steps of the method for predicting a blood pump state as proposed in any of the above method embodiments.

It should be understood that in the embodiments of the present application, the processor 502 may be a central processing unit (CPU), and the processor 502 may also be other types of general-purpose processors, digital signal processors (DSP), application-specific integrated circuits (ASIC), field-programmable gate arrays (FPGA) or other programmable logic devices, discrete gates or transistor logic devices, discrete hardware components, etc. Among them, the general-purpose processor may be a microprocessor or the processor may also be any conventional processor, etc.

It can be understood by a person of ordinary skill in the art that all or part of the processes in the method for implementing the above embodiment can be completed by instructing the relevant hardware through a computer program. The computer program may be stored in a storage medium, which is a computer-readable storage medium. The computer program is executed by at least one processor in the computer system to implement the process steps of the embodiments of the above method.

Therefore, the present invention also provides a storage medium. The storage medium may be a computer-readable storage medium. The storage medium stores a computer program. When the computer program is executed by a processor, the processor is caused to execute the steps of the method for predicting a blood pump state as proposed in any of the above method embodiments.

The storage medium is a physical, non-transient storage medium, such as a USB flash drive, a mobile hard disk, a read-only memory (ROM), a magnetic disk or an optical disk, and other physical storage media that can store program codes. The computer-readable storage medium can be non-volatile or volatile.

A person skilled in the art can realize that the units and algorithm steps of each example described in the embodiments disclosed herein can be implemented by electronic hardware, computer software, or a combination of the two. In order to clearly illustrate the interchangeability of hardware and software, the composition and steps of each example have been generally described in terms of function in the above description. Whether these functions are executed in hardware or software depends on the specific application and design constraints of the technical solution. A person skilled in the art can use different methods to implement the described functions for each specific application, but such implementation should not be considered to be beyond the scope of the present invention.

In the several embodiments provided by the present invention, it should be understood that the disclosed device and method can be implemented in other ways. For example, the device embodiments described above are only illustrative. For example, the division of each unit is only a logical function division, and there may be other division methods in actual implementation. For example, multiple units or components can be combined or integrated into another system, or some features can be omitted or not executed.

The steps in the method of the embodiments of the present invention can be adjusted in order, combined and deleted according to actual needs. The units in the device of the embodiments of the present invention can be combined, divided and deleted according to actual needs. In addition, the functional units in the various embodiments of the present invention can be integrated into a processing unit, or each unit can exist physically separately, or two or more units can be integrated into one unit.

If the integrated unit is implemented in the form of a software functional unit and sold or used as an independent product, it can be stored in a storage medium. Based on this understanding, the technical solution of the present invention, or the part that contributes to the existing technology, or all or part of the technical solution, can be embodied in the form of a software product. The computer software product is stored in a storage medium and includes several instructions for enabling a computer device (which can be a personal computer, terminal, or network device, etc.) to execute all or part of the steps of the method described in each embodiment of the present invention.

In the above embodiments, the description of each embodiment has its own emphasis. For the part that is not described in detail in a certain embodiment, reference may be made to the relevant description of other embodiments.

Obviously, a person skilled in the art can make various changes and modifications to the present invention without departing from the principles and scope of the present invention. In this way, if these modifications and variations of the present invention fall within the scope of the claims of the present invention and its equivalent technology, the present invention is also intended to include these modifications and variations.

The above is only certain specific implementation of the present invention, but the protection scope of the present invention is not limited to this. A person skilled in the art can easily think of various equivalent modifications or replacements within the technical scope disclosed by the present invention. These modifications or replacements should be covered within the protection scope of the present invention. Therefore, the protection scope of the present invention shall be based on the protection scope of the claims.

## Claims

1. A method for predicting a blood pump state, **characterized in that** a pump device is driven by a motor, the method comprising:

    collecting an average phase current of each phase of the motor in a current cycle;
    determining whether the average phase current of each phase of the motor is stable;
    determining whether the average phase current of each phase of the motor changes consistently in response to determining that the average phase current of each phase of the motor is unstable;

determining whether the average phase current of each phase of the motor changes shows a cyclic change in response to determining that the average phase current of each phase of the motor changes consistently; and

determining that a blood pump of the pump device is abnormal in response to determining that the average phase current of each phase of the motor changes shows the cyclic change.

2. The method for predicting a blood pump state according to claim 1, **characterized in that** a tail end of the motor of the pump device is equipped with a Hall sensor, and the collecting of the average phase current of each phase of the motor in the current cycle comprises:

determining a starting point and an end point of the current cycle based on a Hall signal of the Hall sensor; and

determining the average phase current of each phase of the motor based on current sampling values collected in each phase of the motor during the current cycle.

3. The method for predicting a blood pump state according to claim 1, **characterized in that** the method further comprises:

determining whether a number of times the average phase current of each phase of the motor changing inconsistently is greater than a preset number of times threshold in response to determining that the average phase current of each phase of the motor changes inconsistently; and

determining that the pump device has a hardware fault in response to determining that the number of times the average phase current of each phase of the motor changing inconsistently is greater than the preset number of times threshold.

4. The method for predicting a blood pump state according to claim 1, **characterized in that** the determining whether the average phase current of each phase of the motor is stable comprises:

determining whether a current fluctuation value of the average phase current of each phase of the motor within a preset time range is greater than a preset current fluctuation threshold; and

determining that the average phase current of each phase of the motor is unstable in response to determining that the current fluctuation value of the average phase current of each phase of the motor within the preset time range is greater than the preset current fluctuation threshold.

5. The method for predicting a blood pump state according to claim 4, **characterized in that** prior to the determining whether the average phase current of each phase of the motor changes consistently, the method further comprises:

determining whether a speed of the pump device is adjusted within the preset time range in response to determining that the average phase current of each phase of the motor is unstable; and

executing the step of determining whether the average phase current of each phase of the motor changes consistently in response to determining that the speed of the pump device is not adjusted within the preset time range.

6. The method for predicting a blood pump state according to claim 4, **characterized in that** prior to the determining whether the average phase current of each phase of the motor changes shows the cyclic change, the method further comprises:

determining whether a flow fluctuation value of a flow of a pipeline where the pump device is located within the preset time range is greater than a preset flow fluctuation threshold in response to determining that the average phase current of each phase of the motor changes consistently;

determining whether the flow fluctuation value of the flow of the pipeline where the pump device is located within the preset time range is not greater than the preset flow fluctuation threshold, determining whether a bubble signal is detected in the pipeline where the pump device is located within the preset time range in response to determining that the average phase current of each phase of the motor changes consistently; and

executing the step of determining whether the average phase current of each phase of the motor changes shows the cyclic change in response to determining that the bubble signal is not detected in the pipeline where the pump device is located within the preset time range.

7. The method for predicting a blood pump state according to claim 6, **characterized in that** the method further comprises:

determining whether the flow fluctuation value of the flow of the pipeline where the pump device is located within the preset time range is greater than the preset flow fluctuation threshold, determining whether a pressure fluctuation value of the pipeline where the pump device is located within the preset time range is greater than a preset pressure fluctuation threshold in response to determining that the average phase current of each phase of the motor changes consistently;

determining that the pipeline where the pump device is located is bent in response to determining that the pressure fluctuation value of the pipeline where the pump device is located within the preset time range is greater than the preset pressure fluctuation threshold;

determining that the pipeline where the pump device is located is shaking in response to determining that the pressure fluctuation value of the pipeline where the pump device is located within the preset time range is not greater than the preset pressure fluctuation threshold; and

determining that a bubble is flowing in the pipeline where the pump device is located in response to determining that the pipeline where the pump device is located is detected with a bubble signal within the preset time range.

8. The method for predicting a blood pump state according to claim 1, **characterized in that** the method further comprises:

determining whether the average phase current of each phase of the motor is greater than a preset no-load current in response to determining that the average phase current of each phase of the motor is stable;

determining that the blood pump of the pump device has fallen off in response to determining that the average phase current of each phase of the motor is not greater than the preset no-load current;

determining whether a flow of the pipeline where the pump device is located is stable in response to determining that the average phase current of each phase of the motor is greater than the preset no-load current; and

determining that the blood pump of the pump device is normal in response to determining that the flow of the pipeline where the pump device is located is stable.

9. The method for predicting a blood pump state according to claim 1, **characterized in that** the method further comprises:

determining whether a number of turns when the average phase current of each phase of the motor is unstable is greater than a preset number of turns threshold in response to determining that the average phase current of each phase of the motor does not show a cyclic change; and

determining that the blood pump of the motor is in a critical state between normal and abnormal in response to determining that the number of turns when the average phase current of each phase of the motor is unstable is greater than a preset number of turns threshold.

10. A device for predicting a blood pump state, **characterized in that** the device comprises units for executing the method according to claim 1.

11. A computer device for predicting a blood pump state of a pump device that is driven by a motor, the computer device comprising:

a processor;

a memory connected to the processor, the memory including a computer program that, when executed by the processor, causes the processor to:

collect an average phase current of each phase of the motor in a current cycle;

determine whether the average phase current of each phase of the motor is stable;

determine whether the average phase current of each phase of the motor changes consistently in response to determining that the average phase current of each phase of the motor is unstable;

determine whether the average phase current of each phase of the motor changes shows a cyclic change in response to determining that the average phase current of each phase of the motor changes consistently; and

determine that a blood pump of the pump device is abnormal in response to determining that the average phase current of each phase of the motor changes shows the cyclic change.

12. A computer-readable storage medium that stores a computer program for predicting a blood pump state of a pump device that is driven by a motor, the computer program including instructions to:

collect an average phase current of each phase of the motor in a current cycle;

determine whether the average phase current of each phase of the motor is stable;

determine whether the average phase current of each phase of the motor changes consistently in response to determining that the average phase current of each phase of the motor is unstable;

determine whether the average phase current of each phase of the motor changes shows a cyclic change in response to determining that the average phase current of each phase of the motor changes consistently; and
determine that a blood pump of the pump device is abnormal in response to determining that the average phase current of each phase of the motor changes shows the cyclic change.

Collect an average phase current of each phase of the motor in a current cycle $S1$

Determine whether the average phase current of each phase of the motor is stable $S2$

If the average phase current of each phase of the motor is unstable, determining whether the average phase current of each phase of the motor changes consistently $S3$

If the average phase current of each phase of the motor changes consistently, determine whether the average phase current of each phase of the motor changes shows a cyclic change $S4$

If the average phase current of each phase of the motor changes shows the cyclic change, determine that a blood pump of the pump device is abnormal $S5$

FIG. 1

S101

Collect an average phase current of each phase of the motor in a current cycle

S102

Determine whether the average phase current of each phase of the motor is stable

N

Y

S103

Determine whether the average phase current of each phase of the motor is greater than a preset no-load current

Y

N

S104

Determine that the blood pump of the pump device has fallen off

S105

Determine whether a flow of the pipeline where the pump device is located is stable

Y

N

S106

Determine that the blood pump of the pump device is normal

S107

Determine whether a speed of the pump device is adjusted within the preset time range

Y

N

S108

Determine whether the average phase current of each phase of the motor changes consistently

Y

N

S109

Determine whether a number of times the average phase current of each phase of the motor changing inconsistently is greater than a preset number of times threshold

N

Y

S110

Determine that the pump device has a hardware fault

S111

Determine whether a flow fluctuation value of a flow of a pipeline where the pump device is located within the preset time range is greater than a preset flow fluctuation threshold

N

Y

S112

Determine whether a pressure fluctuation value of the pipeline where the pump device is located within the preset time range is greater than a preset pressure fluctuation threshold

N

Y

S113

Determine that the pipeline where the pump device is located is bent

S114

Determine that the pipeline where the pump device is located is shaking

S115

Determine whether a bubble signal is detected in the pipeline where the pump device is located within the preset time range

N

Y

S116

Determine that a bubble is flowing in the pipeline where the pump device is located

S117

Determine whether the average phase current of each phase of the motor changes shows the cyclic change

N

Y

S118

Determine that a blood pump of the pump device is abnormal

S119

Determine whether a number of turns when the average phase current of each phase of the motor is unstable is greater than a preset number of turns threshold

S120

If the number of turns when the average phase current of each phase of the motor is unstable is greater than a preset number of turns threshold, determine that the blood pump of the motor is in a critical state between normal and abnormal

FIG. 2

18

Pump head state prediction device

Collecting unit — 21

First judging unit — 22

Second judging unit — 23

Third judging unit — 24

First determining unit — 25

— 20

FIG. 3

— 500

Computer device

Processor — 502

— 501

— 503

Operating system — 5031

Computer program — 5032

Non-volatile storage medium

Internal memory — 504

Network interface — 505

FIG. 4

Europäisches
Patentamt

European
Patent Office

Office européen
des brevets

## EUROPEAN SEARCH REPORT

**Application Number**

EP 25 16 2203

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2012/142998 A1 (OGAWA DAISUKE [JP] ET AL) 7 June 2012 (2012-06-07) * paragraphs 0094, 0116, 0118, 0136; figures 1, 5, 7 * ----- | 1-12 | INV. F04D13/06 F04D15/02 A61M60/113 A61M60/232 |
| A | US 2012/245681 A1 (CASAS FERNANDO [US] ET AL) 27 September 2012 (2012-09-27) * paragraphs 0022, 0023, 0059; figures 3, 4 * ----- | 1-12 | A61M60/38 A61M60/508 A61M60/90 F04D15/00 |
| A | US 2020/093973 A1 (GANDHI RAHUL SURESH [US] ET AL) 26 March 2020 (2020-03-26) * paragraphs 0005, 0006 * ----- | 1-12 | |

**TECHNICAL FIELDS SEARCHED (IPC)**

F04D
A61M

The present search report has been drawn up for all claims

| Place of search | Date of completion of the search | Examiner |
|---|---|---|
| The Hague | 31 May 2025 | Martin Amezaga, J |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

 

& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 25 16 2203

This annex lists the patent family members relating to the patent documents cited in the above-mentioned European search report.
The members are as contained in the European Patent Office EDP file on
The European Patent Office is in no way liable for these particulars which are merely given for the purpose of information.

31-05-2025

| Patent document cited in search report | Publication date | Patent family member(s) | | Publication date |
|---|---|---|---|---|
| US 2012142998 A1 | 07-06-2012 | EP | 2460545 A1 | 06-06-2012 |
| | | JP | 5810459 B2 | 11-11-2015 |
| | | JP | 2012115619 A | 21-06-2012 |
| | | US | 2012142998 A1 | 07-06-2012 |
| US 2012245681 A1 | 27-09-2012 | AU | 2012207146 A1 | 05-09-2013 |
| | | CA | 2825354 A1 | 26-07-2012 |
| | | CN | 103328018 A | 25-09-2013 |
| | | EP | 2665499 A1 | 27-11-2013 |
| | | KR | 20140040112 A | 02-04-2014 |
| | | US | 2012245681 A1 | 27-09-2012 |
| | | WO | 2012100210 A1 | 26-07-2012 |
| US 2020093973 A1 | 26-03-2020 | AU | 2019342744 A1 | 06-05-2021 |
| | | AU | 2024227114 A1 | 24-10-2024 |
| | | CA | 3112595 A1 | 26-03-2020 |
| | | CN | 112703032 A | 23-04-2021 |
| | | CN | 118526706 A | 23-08-2024 |
| | | EP | 3852833 A1 | 28-07-2021 |
| | | IL | 281557 A | 31-05-2021 |
| | | IL | 314934 A | 01-10-2024 |
| | | JP | 7466529 B2 | 12-04-2024 |
| | | JP | 2022501119 A | 06-01-2022 |
| | | JP | 2024096747 A | 17-07-2024 |
| | | KR | 20210064195 A | 02-06-2021 |
| | | SG | 11202102187Q A | 29-04-2021 |
| | | US | 2020093973 A1 | 26-03-2020 |
| | | US | 2023173251 A1 | 08-06-2023 |
| | | WO | 2020061399 A1 | 26-03-2020 |

EPO FORM P0459

For more details about this annex : see Official Journal of the European Patent Office, No. 12/82